# EUROPÄISCHE PATENTSCHRIFT

(11) **EP 3 417 290 B1**
(45) Veröffentlichungstag und Bekanntmachung des Hinweises auf die Patenterteilung: **02.12.2020**
(21) Anmeldenummer: 17715645.2
(22) Anmeldetag: 27.03.2017
(51) Int. Cl.: G01N 33/543, G01N 29/02

(54) **TESTKIT ZUR BIOANALYTIK UND VERFAHREN ZUR AUSWERTUNG EINES SOLCHEN TESTKITS**
BIOANALYSIS TEST KIT AND METHOD FOR ANALYZING SUCH A TEST KIT
KIT D'ESSAI POUR ANALYSES BIOLOGIQUES ET PROCÉDÉ D'ÉVALUATION D'UN TEL KIT D'ESSAI

(30) Priorität: 31.03.2016 DE 102016205335
(43) Veröffentlichungstag der Anmeldung: 26.12.2018
(73) Patentinhaber: Siemens Aktiengesellschaft, 80333 München (DE)
(72) Erfinder: GIGLER, Alexander Michael, 86836 Untermeitingen (DE); SCHREITER, Matthias, 81379 München (DE); STÜTZ, Evamaria, 81827 München (DE); BUCHHOLZ, Stephan, 81739 München (DE)
(86) Internationale Anmeldenummer: PCT/EP2017/057171
(87) Internationale Veröffentlichungsnummer: WO 2017/167679

(56) Entgegenhaltungen:
- WO-A1-2015/073496
- US-A1- 2007 224 700
- US-A1- 2008 220 980
- US-A1- 2010 227 773

## Beschreibung

Die Erfindung betrifft ein Testkit zur Bioanalytik sowie ein Verfahren zur Auswertung eines solchen Testkits.

Bioanalytische Tests wie beispielsweise Schwangerschafts- oder Suchtmitteltests mittels Teststreifen liefern typischerweise nur qualitative, nicht quantitative, Resultate. Insbesondere bei Tests, welche sich an Endanwender außerhalb eines entsprechend eingerichteten Labors wenden, ist eine eindeutige Referenzskala für eine quantitative Analyse regelmäßig nicht verfügbar.

Auch eine Quantifizierung etwa von Suchtmitteln in Körperflüssigkeiten erfolgt typischerweise nachgelagert in einem Labor. Gerade im Falle von Suchtmitteln sind quantitativ bereits im Feldeinsatz auswertbare Testkits wünschenswert, etwa anhand von Speichelproben bei einer Kontrolle. Insbesondere aufgrund der Vielzahl potentiell relevanter Einflussparameter jedoch bleiben quantitative Messungen im Feldeinsatz, so überhaupt möglich, in ihrer Genauigkeit stets deutlich hinter Labormessungen zurück.

Daher dienen nicht laborgebundene Testkits regelmäßig allenfalls als "schnelle Vorabtests". Typischerweise wird dabei ein Analyt auf einen Teststreifen aufgebracht und etwa das Eintreten einer Verfärbung beobachtet. Eine Quantifizierbarkeit ist bei solchen Testkits kaum möglich.

WO 2015/073496 offenbart analyterfassende Sensor-Vorrichtungen. US 2010/227773 beschreibt eine Vorrichtung und ein Verfahren zur Bestimmung mehrerer Analyte.

Es ist vor diesem Hintergrund Aufgabe der Erfindung, ein verbessertes Testkit sowie ein verbessertes Verfahren zur Erfassung von Messdaten zur Bioanalytik bereitzustellen, welche insbesondere ein zuverlässiges, quantitatives, Testresultat erlauben. Ferner ist es Aufgabe der Erfindung, ein verbessertes Verfahren zur Auswertung eines solchen Testkits sowie eine Auswerteinrichtung zur Auswertung solcher Testkits bereitzustellen.

Diese Aufgabe der Erfindung wird mit einem Testkit mit den in Anspruch 1 angegebenen Merkmalen, einem Verfahren zur Erfassung von Messdaten mit den in Anspruch 8 angegebenen Merkmalen sowie mit einem Verfahren zur Auswertung eines Testkits mit den in Anspruch 9 angegebenen Merkmalen und mit einer Auswerteinrichtung mit den in Anspruch 10 angegebenen Merkmalen gelöst. Bevorzugte Weiterbildungen der Erfindung sind in den zugehörigen Unteransprüchen, der nachfolgenden Beschreibung und der Zeichnung angegeben.

Das erfindungsgemäße Testkit ist ausgebildet zur Bioanalytik, insbesondere für ein Immunassay. Das erfindungsgemäße Testkit weist zumindest einen Messsensor zur quantitativen Detektion einer Substanz sowie zumindest einen bereits definiert mit der Substanz beaufschlagten Referenzsensor auf. Unter einer definierten Beaufschlagung des oder der Referenzsensoren ist die Beaufschlagung mit einer bestimmten Konzentration und/oder Stoffmenge und/oder Masse (absolut oder relativ) zu verstehen. Entsprechend ist unter einer quantitativen Detektion die Detektion einer Konzentration und/oder Stoffmenge und/oder Masse (absolut oder relativ) zu verstehen.

Kerngedanke der Erfindung ist die Idee, eine Referenzskala mittels eines oder mehrerer Referenzsensoren direkt in das Testkit selbst zu implementieren, sodass die Referenzskala stets die äußerlichen Einflüsse auf das Testkit und somit auf dem Messsensor berücksichtigt. Beispielsweise wirken sich Temperatureinflüsse gleichermaßen auf den oder die Referenzsensoren und den Messsensor aus. Ferner altern die Sensoren und daran ggf. angelagerte, zu messende, Substanz in dem erfindungsgemäßen Testkit gleichermaßen, sodass hieraus resultierende Abweichungen sich zugleich auf den Messsensor sowie auf den oder die Referenzsensoren auswirken und folglich stets kompensieren.

Bevorzugt weist das erfindungsgemäße Testkit zumindest zwei, vorzugsweise zumindest drei oder mehr Referenzsensoren auf. Zweckmäßig sind die Referenzsensoren voneinander abweichend mit der Substanz beaufschlagt. Auf diese Weise kann mittels einer Ausgleichsrechnung eine besonders weite Referenzskala für Auslesewerte des Messsensors erhalten werden.

Bei einer besonders bevorzugten Weiterbildung des erfindungsgemäßen Testkits ist oder sind der oder die Referenzsensoren und/oder der oder die Messsensoren mit akustischen Resonatoren, insbesondere mit FBARs, gebildet.

Gerade mit akustischen Resonatoren, insbesondere mit FBARs, gebildete Sensor-Arrays können Targetmolekülen sehr sensitiv detektieren. FBARs sind miniaturisierte akustische Resonatoren, deren Resonanzfrequenz sich durch Anlagerung von Molekülen auf deren Oberfläche hochsensitiv verstimmen lässt. Durch geeignete Funktionalisierungen lassen sich dabei Targetmoleküle selektiv binden und damit gezielt nachweisen.

Beliebig viele FBAR-Resonatoren können in einen Mess- und/oder Referenzsensor integriert werden, wobei die Resonatoren einzeln ansteuerbar und auslesbar bleiben. Dadurch stellt jeder einzelne Resonator eine unabhängige Reaktionsfläche für z.B. lmmunassays dar. Die Resonatoren können insbesondere durch die Bindung von Antikörpern oder von standardisierten Antikörper-Antigen-Komplexen funktionalisiert werden. Weiterhin lassen sich die Resonatoren einfach mittels Kompartimenten, insbesondere auf einem Sensorchip, mit präzise definierten Konzentrationen beaufschlagen. Regelmäßig und ebenfalls besonders vorteilhaft sind akustischen Resonatoren wie an sich bekannt mit piezoelektrischen Materialien gebildet, sodass sie sich einfach, automatisiert und zuverlässig elektrisch ansteuern und auslesen lassen.

Zweckmäßig umfassen bei dem erfindungsgemäße Testkit der zumindest eine Messsensor und der oder die Referenzsensoren je mindestens einen, vorzugsweise mittels Antigenen und/oder mittels Antikörpern, funktionalisierten akustischen Resonator.

Zweckmäßig weist das erfindungsgemäße Testkit je Messsensor zumindest ein Messkompartiment auf, in welchem der zumindest eine Messsensor jeweils eingebracht ist und/oder je Referenzsensor zumindest ein Referenzkompartiment auf, in welchem der zumindest eine Referenzsensor jeweils eingebracht ist. Auf diese Weise ist eine fluiddichte Separation der Mess- oder Referenzsensoren zuverlässig gewährleistet.

In einer vorteilhaften Weiterbildung ist das erfindungsgemäße Testkit ein Schwangerschafts- und/oder Suchtmittel-Testkit. Ein Suchtmittel umfasst im Rahmen dieser Anmeldung insbesondere Betäubungsmittel und/oder Rauschmittel, bei welchen es unzulässig ist, sich unter ihren Einfluss allgemein oder bei bestimmten Tätigkeiten, zumindest ab einem Mindestschwellwert, zu begeben.

Bei dem erfindungsgemäßen Verfahren zur Erfassung von Messdaten für ein Immunassay, wird ein Bioanalytik-Testkit wie zuvor beschrieben herangezogen.

Bei dem erfindungsgemäßen Verfahren zur Auswertung eines Testkits wie zuvor beschrieben wird der Messsensor ausgelesen und ein Messwert für eine Konzentration, eine Stoffmenge oder eine Masse erhalten, indem der Auslesewert des zumindest einen Messsensors unter Heranziehung der Auslesewerte des zumindest einen Referenzsensors entweder einfach skaliert wird oder aber mittels einer die Auslesewerte der Referenzsensoren mit den definierten Beaufschlagungen der Referenzsensoren in Beziehung setzenden Ausgleichsfunktion oder -kurve ein mit dem Auslesewert korrespondierender Messwert erhalten wird. Die erfindungsgemäße Auswerteinrichtung ist ausgebildet zur Ausführung eines Auswertverfahrens wie zuvor beschrieben. Besonders bevorzugt ist die Auswerteinrichtung ein Bestandteil des erfindungsgemäßen Testkits wie zuvor beschrieben.

Nachfolgend wird die Erfindung anhand eines in der Zeichnung dargestellten Ausführungsbeispiels näher erläutert.

Es zeigen:
- Fig. 1: ein Testkit mit einem Kompartiment mit einem mit mehreren akustischen Resonatoren gebildeten Messsensor sowie drei Kompartimenten mit je einem mit mehreren akustischen Resonatoren gebildeten Referenzsensor während der Vorbereitung für den Feldeinsatz schematisch in einer Draufsicht sowie

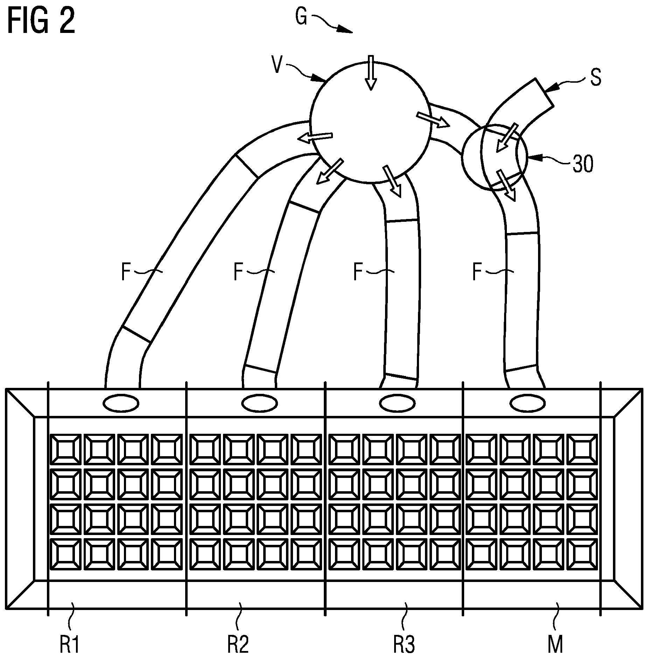

- Fig. 2: das Testkit gemäß Fig. 1 in der Konfiguration während des Feldeinsatzes schematisch in einer Draufsicht.

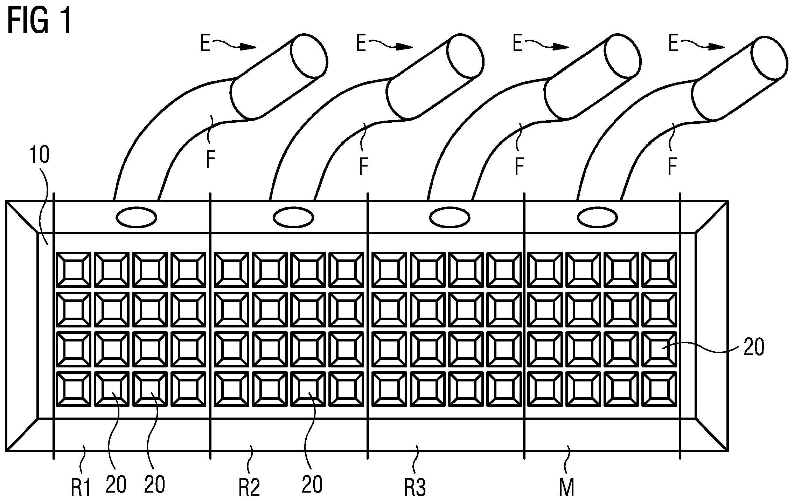

Das in Fig. 1 dargestellte Testkit umfasst einen Sensorchip 10, welcher ein Array von akustischen Resonatoren 20 in Gestalt von FBARs (FBAR (engl.) = *"Film Bulk Acoustic Resonator*") aufweist. In an sich bekannter Weise lässt sich mittels solcher akustischen Resonatoren 20 die Anlagerung von Substanzen aus einer Verstimmung der Resonanzfrequenz detektieren. Im in Fig. 1 dargestellten Fall bilden je 16 Resonatoren einen Sensor aus. Dabei bilden drei solcher mittels Gruppen von Resonatoren gebildete Sensoren jeweils einen Referenzsensor R1, R2, R3 aus und eine vierte Gruppe bildet den eigentlichen Messsensor M.

Die Referenzsensoren R1, R2, R3 und der Messsensor M sind jeweils voneinander fluiddicht getrennt, indem in an sich bekannter Weise auf der Chipoberfläche biokompatible Kompartimente, etwa mit PBO gebildet, mittels eines Waverlevel-Prozesses abgeschieden sind. Jeder der Referenzsensoren R1, R2, R3 sowie der Messsensor M sind dabei in jeweils einem einzelnen Kompartiment angeordnet, an die jeweils fluiddicht eine mittels Spritzguss aus PEEK gefertigte Flusszelle angebunden ist.

Zunächst wird das Testkit wie folgt (im dargestellten Fall durch den Hersteller nach Herstellung des Testkits) auf den Feldeinsatz vorbereitet:
Zunächst werden die akustischen Resonatoren 20 funktionalisiert. Dazu ist jedes Kompartiment der Referenzsensoren R1, R2, R3 und des Messsensors M über die jeweils zugeordnete Flusszelle an eine Fluidzuführung F angebunden. Zunächst wird jedem Einlass E der jeweiligen Fluidzuführung F eine assaykompatible Pufferlösung, im dargestellten Ausführungsbeispiel eine phosphatgepufferte Kochsalzlösung (PBS) zugeführt. Mittels dieser Pufferlösung wird jedes Kompartiment des jeweiligen Referenzsensors R1, R2, R3 und des Messsensors M gespült.

Nachfolgend werden sowohl die Resonatoren 20 der Referenzsensoren R1, R2, R3 als auch die Resonatoren 20 des Messsensors M mit einem Fänger-Antikörper funktionalisiert, d.h. oberflächlich beschichtet.

Nach einem abermaligen Spülschritt jedes Kompartiments des jeweiligen Referenzsensors R1, R2, R3 und des Messsensors M werden nichtspezifische Anlagerungsflächen des Sensorchips 10, d.h. Flächen, welche keine funktionalisierten Flächen der Resonatoren 20 bilden, passiviert. Dazu wird jeweils über den Einlass E der jeweiligen Fluidzuführung F Albumin (hsa/bsa) zugegeben.

Nach einem neuerlichen Spülschritt jedes Kompartiments des jeweiligen Referenzsensors R1, R2, R3 und des Messsensors M wird jedes der Kompartimente der Referenzsensoren R1, R2, R3 (nicht aber das Kompartiment des Messsensors M) mit jeweils einer definierten Konzentration der Targetsubstanz beaufschlagt. Dabei wird jeder der Referenzsensoren R1, R2, R3 mit einer unterschiedlichen Konzentration der Targetsubstanz beaufschlagt. Anschließend werden die den Referenzsensoren R1, R2, R3 zugeordneten Kompartimente sowie das dem Messsensor M zugeordnete Kompartiment mit einer geeigneten Konservierungslösung gefüllt. Der Sensorchip 10 ist somit lagerbar bis zum beabsichtigten Feldeinsatz.

In weiteren, nicht eigens dargestellten Ausführungsbeispielen sind zusätzliche Kompartimente mit weiteren Mess- und Referenzsensoren vorhanden, welche zur Messung weiterer Substanzen dienen. Grundsätzlich können in weiteren nicht gesondert dargestellten und hier nicht im Detail erläuterten Ausführungsbeispielen die Resonatoren mittels Mikrospottern funktionalisiert sein, sodass insbesondere einzelne Resonatoren innerhalb eines Kompartiments für voneinander verschiedene Substanzen funktionalisiert sein können.

Für den Feldeinsatz werden die Zuführungen F mit ihren Einlässen E an einen Verteiler V angeschlossen, welcher einen gemeinsamen Einlass G aufweist. Ferner ist die den Messsensor speisende Zuführung F mit einem 3-Wege-Ventil 30 versehen, mittels welchem entweder der Verteiler V oder ein Probeneinlass S mit dem Kompartiment des Messsensor M fluidisch verbindbar ist. Zunächst wird der Sensorchip 10 im Feldeinsatz aktiviert, indem zunächst die Kompartimente der Referenzsensoren R1, R2, R3 sowie des Messsensors M mit Pufferlösung gespült werden. Somit wird die Konservierungslösung vollständig entfernt.

Durch Stellen des 3-Wege-Ventils 30 wird nun der Messsensor M fluidisch abgekoppelt und es wird dem Messsensor M mittels des Probeneinlasses S die Targetsubstanz zugeführt und so der Messsensor M mit der Targetsubstanz beaufschlagt. Währenddessen werden die den Referenzsensoren R1, R2, R3 zugeordneten Kompartimente weiterhin mit der Pufferlösung gespült. Anschließend wird sämtlichen Kompartimenten der Referenzsensoren R1, R2, R3 sowie des Messsensors M der zum Fänger-Antikörper passende Antigenkomplex zugeführt.

Im Anschluss und zur Messung werden alle Kammern dann wieder zugleich und mit gleicher Strömung mit Puffer gespült und die Messung der Verschiebung der Resonanzfrequenz der FBARs durchgeführt.

Nachfolgend werden die Messwerte des Sensorchips 10 ausgewertet. Dazu werden zunächst die entsprechend der vorhergehenden definierten Beaufschlagung der Referenzsensoren R1, R2, R3 mit den Auslesewerten der Referenzsensoren R1, R2, R3 in Beziehung gesetzt. Im dargestellten Ausführungsbeispiel werden mithin die Konzentrationen der Targetsubstanz, mit welchen die Referenzsensoren R1, R2 und R3 bei der Vorbereitung des Sensorchips 10 beaufschlagt worden sind, mit der Frequenzverschiebung der Resonanzfrequenz der akustischen Resonatoren 20 der jeweiligen Referenzsensoren R1, R2 und R3 in Beziehung gesetzt. Der in sehr guter Näherung lineare Zusammenhang wird mit einer Ausgleichsgerade abgebildet. Mittels der Ausgleichsgerade kann nun eine Frequenzverschiebung des Messsensors M in eine Konzentration der Targetsubstanz umgerechnet werden.

In einem weiteren Ausführungsbeispiel, welches dem zuvor erläuterten Ausführungsbeispiel entspricht, ist die Beaufschlagung eines Referenzsensors R1, R2, R3 mit einer Konzentration gewählt, welche einem etwa für eine Fahrzeugführung, eine Maschinenbedienung oder einem sonst rechtlich bedeutsamen Grenzwert entspricht. Vergleicht man die Frequenzverschiebung des Messsensors M mit der Frequenzverschiebung dieses Referenzsensors, so lässt sich direkt auf eine Konzentration der Targetsubstanz jenseits des Grenzwerts schließen.

Bei den zuvor erläuterten Ausführungsbeispielen kann verborgen bleiben, welche Kompartimente wie im Detail funktionalisiert sind. Folglich ist eine Manipulation des Sensorchips 10 wirksam verhindert oder aber eine Manipulation kann leicht erkannt werden.

## Patentansprüche

1. Testkit, ausgebildet zur Bioanalytik insbesondere für ein Immunassay, welches zumindest einen Messsensor (M) zur quantitativen Detektion einer Substanz sowie zumindest einen bereits definiert mit der Substanz beaufschlagten Referenzsensor (R1, R2, R3) umfasst und bei welchem der oder die Referenzsensoren (R1, R2, R3) und/oder der oder die Messsensoren (M) mit akustischen Resonatoren (20), insbesondere mit FBARs, gebildet ist oder sind und welches je Referenzsensor (R1, R2, R3) zumindest ein Referenzkompartiment aufweist, in welchem der zumindest eine Referenzsensor (R1, R2, R3) jeweils eingebracht ist.

2. Testkit nach einem der vorhergehenden Ansprüche, bei welchem der zumindest eine Referenzsensor (R1, R2, R3) mit je einer definierten Konzentration oder Stoffmenge oder Masse der Substanz beaufschlagt ist.

3. Testkit nach einem der vorhergehenden Ansprüche, welches zumindest zwei, vorzugsweise zumindest drei oder mehr Referenzsensoren (R1, R2, R3) umfasst, welche zweckmäßig voneinander abweichend mit der Substanz beaufschlagt sind.

4. Testkit nach einem der vorhergehenden Ansprüche, bei welchem der zumindest eine Messsensor (M) und der oder die Referenzsensoren (R1, R2, R3) je mindestens einen, vorzugsweise mittels Antigenen und/oder mittels Antikörpern, funktionalisierten akustischen Resonator umfassen.

5. Testkit nach einem der vorhergehenden Ansprüche, welches je Messsensor (M) zumindest ein Messkompartiment aufweist, in welchem der zumindest eine Messsensor (M) jeweils eingebracht ist.

6. Testkit nach einem der vorhergehenden Ansprüche, welches ein Schwangerschafts- und/oder Suchtmittel-Testkit ist.

7. Verfahren zur Erfassung von Messdaten für ein Immunassay, bei welchem ein Bioanalytik-Testkit (10) nach einem der vorhergehenden Ansprüche herangezogen wird.

8. Verfahren zur Auswertung eines Testkits (10) nach einem der vorhergehenden Ansprüche, bei welchem der Auslesewert des zumindest einen Messsensors (M) ausgelesen wird und ein Messwert für eine Konzentration, eine Stoffmenge oder eine Masse erhalten wird, indem der Auslesewert des zumindest einen Messsensors (M) unter Heranziehung der Auslesewerte des zumindest einen Referenzsensors (R1, R2, R3) skaliert wird oder mittels einer die Auslesewerte der Referenzsensoren (R1, R2, R3) mit den definierten Beaufschlagungen der Referenzsensoren (R1, R2, R3) in Beziehung setzenden Ausgleichsfunktion ein mit dem Auslesewert des Messsensors korrespondierender Messwert erhalten wird.

9. Testkit nach einem der Ansprüche 1 bis 6 mit einer Auswerteinrichtung, ausgebildet zur Ausführung eines Auswertverfahrens nach Anspruch 8.

## Claims

1. Test kit, embodied for bioanalysis, in particular for an immunoassay, comprising at least one measurement sensor (M) for quantitative detection of a substance and at least one reference sensor (R1, R2, R3), to which the substance has already been supplied in a defined manner, and wherein the reference sensor or sensors (R1, R2, R3) and/or the measurement sensor or sensors (M) is/are formed with acoustic resonators (20), in particular FBARs, and comprising at least one reference compartment per reference sensor (R1, R2, R3), the at least one reference sensor (R1, R2, R3) respectively being introduced therein.

2. Test kit according to any one of the preceding claims, wherein the at least one reference sensor (R1, R2, R3) is supplied with a defined concentration or substance amount or mass of the substance in each case.

3. Test kit according to any one of the preceding claims, comprising at least two, preferably at least three or more reference sensors (R1, R2, R3), the substance expediently being supplied to a different extent to each of said sensors.

4. Test kit according to any one of the preceding claims, wherein the at least one measurement sensor (M) and the reference sensor or sensors (R1, R2, R3) each comprise at least one acoustic resonator, which preferably has been functionalized by means of antigens and/or antibodies.

5. Test kit according to any one of the preceding claims, comprising at least one measurement compartment per measurement sensor (M), the at least one measurement sensor (M) respectively being introduced therein.

6. Test kit according to any one of the preceding claims, said test kit being a pregnancy and/or drugs test kit.

7. Method for acquiring measurement data for an immunoassay, wherein use is made of a bioanalysis test kit (10) according to any one of the preceding claims.

8. Method for evaluating a test kit (10) according to any one of the preceding claims, wherein the readout value of the at least one measurement sensor (M) is read and a measurement value is obtained for a concentration, a substance amount or a mass by virtue of the readout value of the at least one measurement sensor (M) being scaled using the readout values of the at least one reference sensor (R1, R2, R3) or a measurement value corresponding to the readout value of the measurement sensor being obtained by means of a compensation function that relates the readout values of the reference sensors (R1, R2, R3) to the defined amounts supplied to the reference sensors (R1, R2, R3).

9. Test kit according to any one of Claims 1 to 6, comprising an evaluation device which is embodied to implement an evaluation method according to Claim 8.

## Revendications

1. Kit de test, conçu pour une analyse biologique, en particulier pour un immunodosage, qui comprend au moins un capteur de mesure (M) destiné à la détection quantitative d'une substance, de même qu'au moins un capteur de référence (R1, R2, R3) déjà défini, sollicité avec la substance, et dans lequel le ou les capteurs de référence (R1, R2, R3) et/ou le ou les capteurs de mesure (M) sont réalisés avec des résonateurs acoustiques (20), en particulier avec des FBAR ou représentent lesdits résonateurs, et dont chaque capteur de référence (R1, R2, R3) présente au moins un compartiment de référence dans lequel est respectivement incorporé ledit au moins un capteur de référence (R1, R2, R3).

2. Kit de test selon l'une quelconque des revendications précédentes, dans lequel ledit au moins un capteur de référence (R1, R2, R3) est sollicité avec respectivement une concentration ou une quantité de matière ou une masse définie de la substance.

3. Kit de test selon l'une quelconque des revendications précédentes, qui comprend au moins deux, de préférence au moins trois capteurs de référence (R1, R2, R3) ou plus qui sont sollicités avec la substance en étant disposés de manière appropriée à l'écart les uns des autres.

4. Kit de test selon l'une quelconque des revendications précédentes, dans lequel ledit au moins un capteur de mesure (M) et le ou les capteurs de référence (R1, R2, R3) comprennent respectivement au moins un résonateur acoustique fonctionnalisé de préférence au moyen d'antigènes et/ou au moyen d'anticorps.

5. Kit de test selon l'une quelconque des revendications précédentes, dont chaque capteur de mesure (M) présente au moins un compartiment de mesure dans lequel ledit au moins un capteur de mesure (M) est respectivement incorporé.

6. Kit de test selon l'une quelconque des revendications précédentes, qui représente un kit de test de grossesse et/ou de stupéfiant.

7. Procédé destiné à l'enregistrement de données de mesure pour un immunodosage, dans lequel on fait appel à un kit de test (10) destiné à une analyse biologique selon l'une quelconque des revendications précédentes.

8. Procédé destiné à l'évaluation d'un kit de test (10) selon l'une quelconque des revendications précédentes, dans lequel on lit la valeur de lecture dudit au moins un capteur de mesure (M) et on obtient une valeur de mesure pour une concentration, une quantité de matière ou une masse ; dans lequel on procède à l'étalonnage de la valeur de lecture dudit au moins un capteur de mesure (M) en se référant aux valeurs de lecture dudit au moins un capteur de référence (R1, R2, R3) ou on obtient une valeur de mesure qui correspond à la valeur de lecture du capteur de mesure au moyen d'une fonction de comparaison qui met en rapport les valeurs de lecture des capteurs de référence (R1, R2, R3) avec les sollicitations définies des capteurs de référence (R1, R2, R3).

9. Kit de test selon l'une quelconque des revendications 1 à 6, comprenant un mécanisme d'évaluation qui est conçu pour la mise en œuvre d'un procédé d'évaluation selon la revendication 8.
